# EUROPEAN PATENT APPLICATION

(11) **EP 1 321 152 A1**
(43) Date of publication of application: **25.06.2003**
(21) Application number: 01205095.1
(22) Date of filing: 21.12.2001
(51) Int. Cl.: A61K 38/48, A61P 39/02

(54) **Antidote for org 31540/SR 90107A**

(71) Applicant: Akzo Nobel N.V., 6824 BM Arnhem (NL); SANOFI-SYNTHELABO, 75635 Paris Cédex 13 (FR)
(72) Inventor: Amsterdam, Ronald, Gijsbertus, Maria, 5340 BH Oss (NL); Aken, Benien, A, 5340 BH Oss (NL); Meuleman, Dirk, Gerrit, 5340 BH Oss (NL)
(74) Representative: Kraak, Hajo

(57) **Abstract**

The invention relates to the use of recombinant activated factor VII as an antidote for counteracting the effects of ORG 31540 / SR 90107A in patients in need of such treatment.

## Description

The invention relates to the use of an antidote in couteracting / neutralizing the anticoagulant effect induced by an anticoagulant (oligo)saccharide, in particular ORG 31540 / SR 90107A. Further the invention relates to a kit of parts comprising a pharmaceutical composition for said use.

It is well known to treat and prevent thrombin-mediated and thrombin-associated diseases by the use of anticoagulant saccharides, such as unfractionated heparin (UFH), low molecular weight heparins (LMWH) or certain synthetic oligosaccharides. For example, ORG 31540 / SR 90107A (described in Chemical Synthesis to Glycosaminoglycans, Supplement to Nature 1991, *350,* 30-33), is an effective anticoagulant pentasaccharide with a high affinity for antithrombin III (AT). Its binding to AT results in a highly potent and selective inhibition of coagulation factor Xa, to be effective *e*.*g*. in prevention and treatment of acute thrombotic diseases.
For clinical safety, to control major bleeding complications *e.g.* caused by overdosing of the anticoagulant, the therapy with an anticoagulant generally requires the availability of an antidote. This is a medicament that neutralizes / inactivates or reverses / counteracts the action of the anticoagulant. For heparin protamine sulfate is the known antidote. Protamine sulfate may also be used as an antidote for LMWH, however less successfully. For smaller oligosaccharides, in particular for pentasaccharides, protamine sulfate is ineffective as an antidote. Until now no effective antidotes have been reported for Org 31540 / SR 90107A.

Surprisingly, it has now been found that recombinant activated factor VII (rVIIa, NovoSeven®) may effectively be used as an antidote for counteracting the effects of Org 31540 / SR 90107A in patients in need of such treatment.
On the basis of theoretical considerations (based on results reported by PW Friederich et al, Ability of recombinant factor VIIa to generate thrombin during inhibition of tissue factor in human subjects. Circulation 2001;103:2555-2559, in which they assessed the amount of factor X split products) it was not to be expected that administration of rVIIa may normalize or counteract the anticoagulant activity of Org 31540 / SR 90107A in free-flowing blood. *In vitro* data, such as presented by T Lisman *et al*. in Blood, Abstractbook of ASH Dec. 2001, are not predictive of the potential of rVIIa *in vivo.*

The procoagulant NovoSeven® (Novo Nordisk Pharmaceuticals Ltd) is Recombinant Coagulation Factor VIIa, and structurally very related to plasma-derived activated Factor VII (Human). NovoSeven® is known as a product administered only in centres specialising in the treatment of patients with coagulation factor VIII or IX inhibitors.

Haemorrhage is the major clinical sign of an overdose of an anticoagulant. In the event of major bleeding, administration of the anticoagulant must be stopped. According to the present invention, unwanted high plasma levels of the anticoagulant Org 31540 / SR 90107A and bleeding can be treated by use of NovoSeven® in a dose of 30-90 µg/kg. In the clinical trial setting of this invention a high dose of Org 31540 / SR 90107A was neutralized by 90 µg/kg. If in normal clinical situations an antidote is necessary, usually lower doses will be required. Therefore, the dose of NovoSeven® is preferably in the lower dosage range (30-50 µg/kg). The antidote regimen must be continued until bleeding stops. The patient treated is a mammal, in particular a human.

The antidote may be used in the form as provided by the manufacturer, but may also be presented as another pharmaceutical composition comprising the active ingredient rVIIa, with other or additional pharmaceutically acceptable auxiliaries and optionally other therapeutic agents. The term "acceptable" means being compatible with the other ingredients of the composition and not deleterious to the recipients thereof.

Another aspect of the invention is a kit of parts comprising (a) a pharmaceutical composition comprising as active ingredient recombinant activated factor VII together with pharmaceutically acceptable auxiliaries and (b) a pharmaceutical composition comprising ORG 31540 / SR 90107A together with pharmaceutically acceptable auxiliaries. The kit is preferably provided in combination with suitable packaging material including instructions for the use of the pharmaceutical compositions for the use as hereinbefore described.

The pharmaceutical composition (a) may be presented in unit-dose or multi-dose containers, e.g. injection liquids in predetermined amounts, for example in sealed vials and ampoules, and may also be stored in a freeze dried (lyophilized) condition requiring only the addition of sterile liquid carrier, e.g. water, prior to use. The pharmaceutical composition (a) may also be presented in the form of a veterinary composition for use in animals, such compositions may be prepared by methods conventional in the art.
The pharmaceutical composition (b) is a composition for parenteral administration of the ORG 31540 / SR 90107A and may be presented in unit-dose or multi-dose containers, e.g. injection liquids in predetermined amounts, for example in sealed vials and ampoules, and may also be stored in a freeze dried (lyophilized) condition requiring only the addition of sterile liquid carrier, e.g. water, prior to use.
Mixed with such pharmaceutically acceptable auxiliaries and liquids, e.g. as described in the standard reference, Gennaro et al., Remington's Pharmaceutical Sciences, (18th ed., Mack Publishing Company, 1990, see especially Part 8: Pharmaceutical Preparations and Their Manufacture), ORG 31540 / 90107A can be applied as a fluid composition, an injection preparation, in the form of a solution, suspension or emulsion. Aqueous suspensions, isotone saline solutions and sterile injectable solutions may be used, containing pharmaceutically acceptable dispersing agents and/or wetting agents, such as propylene glycol or butylene glycol. The preferred pharmaceutical composition (b) is an isotone saline solution of ORG 31540 / 90107A. The pharmaceutical composition (b) may also be presented in the form of a veterinary composition, such compositions may be prepared by methods conventional in the art.

The invention is further illustrated by the following example. This should not be considered to be limiting in any way.

### EXAMPLE

**Introduction:** The pentasaccharide fondaparinux (ORG 31540 / 90107A, Arixtra®), a synthetic selective factor Xa inhibitor, has proved beneficial for treatment of thrombotic disorders. As anticoagulant treatment may be complicated by bleeding, the efficacy of recombinant activated factor VII (rVIIa) was tested in neutralizing the anticoagulant effect induced by a high dose of fondaparinux in healthy volunteers.

**Methods and results:** The study was a randomized, partially double-blind, placebo-controlled, three-parallel group trial. Male volunteers were randomized to receive a single subcutaneous administration of either fondaparinux (10 mg) followed by an intravenous bolus injection of rVIIa (90 µg/kg, n=8); or fondaparinux followed by an intravenous bolus injection of placebo (n=4); or saline followed by rVIIa (n=4). Placebo and rVIIa were given in a double-blind fashion two hours after fondaparinux or saline.
Administration of rVIIa after pretreatment with fondaparinux resulted in increases of the level of the thrombin generation marker prothrombin fragment 1.2 (F1+2) and shortening of the PT and aPTT. These in vivo data were supported by the results of two ex vivo thrombin generation tests. The injection with rVIIa, after the treatment with fondaparinux, resulted in a marked transient thrombin generation, reflected by the reduction of the time to fibrin polymerization in the thrombin generation assay and by the induction of endogenous thrombin potential (ETP).

**Conclusion:** This trial demonstrates that injection with rVIIa substantially reverses thrombin generation inhibition by fondaparinux. This result suggests that rVIIa is an effective and safe antidote for fondaparinux.

## Claims

1. A use of recombinant activated factor VII as an antidote for counteracting the effects of ORG 31540 / SR 90107A in patients in need of such treatment.

2. The use of claim 1 wherein recombinant activated factor VII is used in a dose of 30 - 90 µg/kg.

3. A use of recombinant activated factor VII for the preparation of a medicament for counteracting the effects of ORG 31540 / SR 90107A in patients in need of such treatment.

4. The use of claim 3, wherein the dose of recombinant activated factor VII is 30 - 90 µg/kg.

5. A method of treatment wherein the effects of ORG 31540 / SR 90107A are counteracted, comprising the administration of a dose of 30 - 90 µg/kg of recombinant activated factor VII to a patient in need of such treatment.

6. A kit of parts comprising a pharmaceutical composition (a) comprising as active ingredient recombinant activated factor VII together with pharmaceutically acceptable auxiliaries and a pharmaceutical composition (b) comprising ORG 31540 / SR 90107A together with pharmaceutically acceptable auxiliaries.
